# EUROPÄISCHE PATENTANMELDUNG

(11) **EP 4 714 618 A1**
(43) Veröffentlichungstag der Anmeldung: **25.03.2026**
(21) Anmeldenummer: 25202402.1
(22) Anmeldetag: 16.09.2025
(51) Int. Cl.: B25J 15/06

(54) **AUTOMATISIERTES INSTRUMENTENVEREINZELUNGSSYSTEM UND VERFAHREN**

(30) Priorität: 19.09.2024 DE 102024126919
(71) Anmelder: Aesculap AG, 78532 Tuttlingen (DE)
(72) Erfinder: JANSEN-TROY, Arne, 78333 Stockach (DE); EBERHARDT-FONSECA, André-Manuel, 70186 Stuttgart (DE); MAAS, Allan, 78467 Konstanz (DE); KOLLER, Tobias, 78532 Tuttlingen (DE)
(74) Vertreter: Tergau & Walkenhorst Intellectual Property GmbH

(57) **Zusammenfassung**

Greifsystem zum Greifen und Positionieren von medizinischen Instrumenten, umfassend:
a) eine Greifvorrichtung, die einen Elektromagneten umfasst und zur Aufnahme von magnetisierbaren Instrumenten aus einem Instrumentenbehälter mittels des Elektromagneten eingerichtet und vorgesehen ist;
b) eine Adaptervorrichtung (2), die dazu eingerichtet und vorgesehen ist, die Greifvorrichtung (1) an einem Steuersystem (3), bevorzugt einem robotischen System (3), anzubringen;
c) eine Haltevorrichtung, wobei der Elektromagnet mittels der Haltevorrichtung an der Adaptervorrichtung angebracht ist;
d) wobei die Haltevorrichtung eine Aufnahme, insbesondere einen Konus, zur Aufnahme des Elektromagneten umfasst;
und
wobei die Haltevorrichtung (18) derart eingerichtet ist, dass der Elektromagnet (4) zwischen einer ersten Position, bei der der Elektromagnet (4) in der Aufnahme (11) aufgenommen ist und einer zweiten Position, bei der der Elektromagnet (4) aus der Aufnahme (11) ausgefahren ist, verfahrbar ist.

## Beschreibung

### Technisches Gebiet

Die vorliegende Erfindung betrifft ein Greifsystem zum Greifen und Positionieren von medizinischen Instrumenten und ein Verfahren zur Entnahme von chirurgischen Instrumenten aus einem Instrumentenbehälter.

### Hintergrund

Im Stand der Technik sind verschiedene Handhabungen und Verarbeitungen von chirurgischen Instrumenten innerhalb von Aufbereitungseinheiten für Medizinprodukte (AEMP) bekannt. Trotz erheblicher Fortschritte in der Automatisierungstechnik und Robotik sind die Prozesse, die mit der Reinigung, Sterilisation und Vorbereitung dieser Instrumente für den erneuten Einsatz verbunden sind, jedoch weiterhin mit Herausforderungen behaftet. Zu den bestehenden Problemen zählen insbesondere die manuelle Handhabung von Sterilgut-Containern und Siebkörben, die nicht nur zeitaufwendig, sondern auch mit einem erheblichen Risiko für physische Belastungen und Verletzungen des Personals verbunden ist. Das Heben und Tragen schwerer Lasten sowie das präzise Handling der Instrumente stellen eine ergonomische Herausforderung dar und können zu langfristigen Gesundheitsschäden führen. Darüber hinaus besteht die Schwierigkeit, eine effiziente und sichere Interaktion zwischen dem Greifsystem und den zu handhabenden Objekten zu gewährleisten, insbesondere wenn es um die Vereinzelung und das Greifen von Instrumenten aus chaotischen Zuständen geht. Ein solcher chaotischer Zustand entsteht beispielsweise, wenn Instrumente als Schüttgut, das heißt insbesondere zufällig geschüttet und nicht willentlich angeordnet, in einen Sterilgut Behälter gegeben werden. Die Notwendigkeit, die Betriebssicherheit zu erhöhen und gleichzeitig die Wartung und den Austausch von Komponenten zu vereinfachen, stellt eine weitere Herausforderung dar.

### Zusammenfassende Beschreibung der Erfindung

Es ist deshalb eine der vorliegenden Erfindung zu Grunde liegende Aufgabe, die Nachteile von Greifvorrichtungen zum Greifen und Positionieren von medizinischen Instrumenten im Stand der Technik zu überwinden. Insbesondere ist es eine der vorliegenden Erfindung zu Grunde liegende Aufgabe, Greifvorrichtungen und entsprechende Verfahren bereitzustellen, die eine verbesserte Handhabung und Verarbeitung von chirurgischen Instrumenten erlauben, um insbesondere die Effizienz und Sicherheit in der AEMP zu steigern und das Risiko für das Personal zu minimieren.

Diese und andere Probleme werden durch den Gegenstand der beigefügten unabhängigen Ansprüche gelöst.

Bevorzugte Ausführungsformen können den abhängigen Ansprüchen entnommen werden und darüber hinaus aus der folgenden Beschreibung, insbesondere unter Berücksichtigung verschiedener Ausführungsformen, wie sie in den beigefügten Ansprüchen behandelt und beschrieben sind.

Dabei bietet die vorliegende Erfindung insbesondere den Vorteil, den Sortierprozess der medizinischen Instrumente aus einem Instrumentenbehälter, wie bspw. einem Siebkorb, heraus so zu gestalten, dass übereinander gestapelte Instrumente separat voneinander gegriffen werden können. Zudem können mittels des erfindungsgemäßen Greifsystems die Instrumente jeweils an der gewünschten Stelle abgelegt werden, wodurch unnötige Prozessschritte vermieden werden können, bzw. nachfolgende Prozessschritt nicht behindert werden.

Auf besonders vorteilhafte Weise erlaubt das erfindungsgemäße Verfahren, dass chaotisch angeordnete, insbesondere unreine, chirurgische Instrumente aus dem Siebkorb randomisiert entnommen werden und anschließend definiert abgelegt werden können. Anschließend kann der Instrumentenbehälter, also bspw. der Siebkorb oder Reinigungskorb, weiteren Prozessschritten, bspw. mittels Förderbänder, Automated Guided Vehicles (AGVs), Shuttlesystemen etc. den Reinigungs- und Desinfektionsgeräten (RDGs) zugeführt werden.

Die Ausführungsformen, Merkmale und Kombinationen von Merkmalen, wie sie hier in Verbindung mit der Erfindung beschrieben werden, sowie die Kombination von Merkmalen, wie sie in den beigefügten Ansprüchen angegeben sind, aber auch jede Kombination von Merkmalen, wie sie in Verbindung mit den Ausführungsformen erwähnt und beschrieben wird, gelten als hierin offenbart, zumindest jedoch als durch einen Fachmann herleitbar. Insbesondere kann jedes Merkmal und jede Kombination von Merkmalen in den hier beschriebenen Ausführungsformen beispielsweise in einer anderen Kombination, insbesondere in einer anderen Anspruchskategorie, beansprucht werden, mindestens weil die fachkundige Person erkennen wird, dass jede einzelne Kombination der hier genannten Merkmale geeignet ist, zur Lösung des zugrunde liegenden Problems beizutragen.

Des Weiteren können jedes Merkmal und jede Kombination von Merkmalen in den Ansprüchen und in der untenstehenden Beschreibung unabhängig von dem jeweils beanspruchten Gegenstand, unabhängig von Anspruchsabhängigkeiten und Rückverweisen sowie unabhängig von der Anspruchskategorie, in der das Merkmal beansprucht wird, verwendet und separat beansprucht werden. Beispielsweise kann in einer willkürlichen Kombination, die aus einem oder mehreren Ansprüchen ausgewählt ist, eine oder mehrere Ausführungsformen gemäß der untenstehenden Beschreibung und/oder den beigefügten Abbildungen vorgesehen sein.

Die oben beschriebenen Aufgaben werden erfindungsgemäß gelöst durch ein Greifsystem zum Greifen und Positionieren von medizinischen Instrumenten, umfassend:
a) eine Greifvorrichtung, die einen Elektromagneten umfasst und zur Aufnahme von magnetisierbaren Instrumenten aus einem Instrumentenbehälter mittels des Elektromagneten eingerichtet und vorgesehen ist;
b) eine Adaptervorrichtung, die dazu eingerichtet und vorgesehen ist, die Greifvorrichtung an einem Steuersystem, bevorzugt an einem robotischen System, anzubringen;
c) eine Haltevorrichtung, wobei der Elektromagnet mittels der Haltevorrichtung an der Adaptervorrichtung angebracht ist;
d) wobei die Haltevorrichtung eine Aufnahme, insbesondere einen Konus, zur Aufnahme des Elektromagneten umfasst;
   und
wobei die Haltevorrichtung derart eingerichtet ist, dass der Elektromagnet zwischen einer ersten Position, bei der der Elektromagnet in der Aufnahme aufgenommen ist und einer zweiten Position, bei der der Elektromagnet aus der Aufnahme ausgefahren ist, verfahrbar ist.

Die oben beschriebenen Aufgaben werden ebenfalls erfindungsgemäß gelöst durch ein erfindungsgemäßes Verfahren zur Entnahme von chirurgischen Instrumenten aus einem Instrumentenbehälter, insbesondere einem Siebkorb, und insbesondere zur Vereinzelung und Handhabung der chirurgischen Instrumente. Ein solches erfindungsgemäßes Verfahren umfasst wenigstens einen Schritt des Bereitstellens eines Greifsystems und
wahlweise einen Schritt a) des Aktivierens des Elektromagneten;
einen Schritt b) des Verfahrens des Elektromagnets aus einer ersten Position, bei der der Elektromagnet in der Aufnahme aufgenommen ist, in eine zweite Position, bei der der Elektromagnet aus der Aufnahme ausgefahren ist;
einen Schritt c) des Aufnehmens eines magnetisierbaren Instruments mittels des Elektromagneten in der zweiten Position;
einen Schritt d) des Verfahrens des Elektromagnets aus der zweiten Position, bei der der Elektromagnet aus der Aufnahme ausgefahren ist, in die erste Position, bei der der Elektromagnet in der Aufnahme aufgenommen;
wahlweise einen Schritt e) des Deaktivierens des Elektromagnets;
wobei das Greifsystem bevorzugt ein Greifsystem nach der vorliegenden Erfindung ist.

Es sei im Zusammenhang mit dem erfindungsgemäßen Verfahren auch darauf hingewiesen, dass die angegebenen Schritte nicht zwangsläufig in der angegebenen Reihenfolge durchgeführt werden müssen. Die angegebenen Schritte können in jeder anderen geeigneten Reihenfolge oder auch gleichzeitig durchgeführt werden.

Allerdings kann die oben angegebene Reihenfolge für bestimmte Ausführungsformen des erfindungsgemäßen Verfahrens vorteilhaft sein. Gleichzeitig kann zum Beispiel ein Schritt a) des Aktivierens des Elektromagneten vor oder nach einem Schritt b) des Verfahrens des Elektromagnets aus einer ersten Position, bei der der Elektromagnet in der Aufnahme aufgenommen ist, in eine zweite Position, bei der der Elektromagnet aus der Aufnahme ausgefahren ist, erfolgen, sodass dann in einem Schritt c) das Aufnehmen eines magnetisierbaren Instruments mittels des Elektromagneten in der zweiten Position möglich ist. Auch kann ein Schritt b) des Verfahrens des Elektromagnets aus einer ersten Position, bei der der Elektromagnet in der Aufnahme aufgenommen ist, in eine zweite Position, bei der der Elektromagnet aus der Aufnahme ausgefahren ist, vor einem Schritt c) und sodann einem Schritt d) durchgeführt werden. Das heißt, dass der Elektromagnet ausgefahren ist, und dann ein Instrument gegriffen wird, um anschließend den Elektromagneten mit gegriffenem Instrument in die erste Position in die Aufnahme zurückzufahren. Es ist jedoch beispielsweise ebenso denkbar, dass sich das Greifsystem bereits in einer zweiten Position, bei der der Elektromagnet aus der Aufnahme ausgefahren ist, befindet, und sogleich in einem Schritt b) ein magnetisierbares Instrument mittels des Elektromagneten aufgenommen werden kann, oder wahlweise in einem Schritt c) der Elektromagnet aus der zweiten Position, bei der der Elektromagnet aus der Aufnahme ausgefahren ist, in die erste Position, bei der der Elektromagnet in der Aufnahme aufgenommen ist gebracht wird. Der Fachmann wird von daher unmittelbar anerkennen, dass verschiedene Reihenfolgen der erfindungsgemäßen Verfahrensschritte, auf vorteilhafte Weise erfindungsgemäß sind.

Dies ist insbesondere vorteilhaft, da die Flexibilität und Effizienz des Greifsystems hierdurch signifikant erhöht werden.

Ein Schritt a) des Aktivierens des Elektromagneten ist insbesondere dann erforderlich, wenn der Elektromagnet zuvor deaktiviert wurde. Im einfachsten Fall, wird der Elektromagnet aktiviert, ein Instrument gegriffen und zum Ablegen des Instruments wird der Elektromagnet wieder deaktiviert, so dass für jeden Greifzyklus zunächst ein erneutes Aktivieren des Elektromagneten zielführend ist. Es liegt jedoch ebenfalls im Rahmen der Erfindung den Elektromagnet dauerhaft aktiviert zu lassen und ein Ablegen der Instrumente durch ein erfindungsgemäßes Abstreifen, bei aktiviertem Elektromagneten, zu erreichen. In einem solchen Fall kann ein erneutes Aktivieren des Elektromagneten unterbleiben.

Die Möglichkeit, den Elektromagneten zwischen zwei Positionen zu verfahren, ermöglicht es, dass das System sowohl für das präzise Greifen als auch für das sichere Positionieren der medizinischen Instrumente optimal eingesetzt werden kann. Dies trägt dazu bei, die Handhabung der Instrumente zu automatisieren und die Arbeitsbelastung des Personals zu reduzieren, während gleichzeitig die Präzision und Zuverlässigkeit der Prozesse innerhalb der AEMP verbessert werden. Darüber hinaus wird durch die Verwendung eines Elektromagneten das Risiko einer Kontamination minimiert, da der direkte Kontakt zwischen den Instrumenten und dem Bedienpersonal verringert wird. Die Integration in ein robotisches System erweitert zudem die Einsatzmöglichkeiten des Greifsystems, was eine Anpassung an verschiedene Arbeitsumgebungen und Aufgabenstellungen ermöglicht. Dabei können Instrumentenbehälter, insbesondere in der Form von Sterilgut-Containern, bspw. einem Siebkorb oder ähnlichem mit dem erfindungsgemäßen Greifsystem gehandhabt werden und Instrumente aus dem Instrumentenbehälter einzeln entnommen werden.

In dem erfindungsgemäßen Verfahren bzw. bei dem erfindungsgemäßen Greifsystem ist in der ersten Position der Elektromagnet innerhalb der Aufnahme, speziell einem Konus, aufgenommen. Diese Konfiguration ist besonders vorteilhaft geeignet, die magnetisierbaren Instrumente definiert zu positionieren. Insbesondere kann der Elektromagnet in der Aufnahme so positioniert und gegen ungewollte Bewegungen stabilisiert werden, dass das unbeabsichtigte Lösen oder Herunterfallen der gegriffenen Instrumente weitestgehend verhindert wird, auch wenn sich der Roboterarm des robotischen Systems bewegt. Erfindungsgemäß ist es bevorzugt, dass in der ersten Position die Aufnahmefläche oder Magnetfläche des Elektromagneten etwa plan mit den Kanten der Aufnahme, bzw. des Konus, ist.

Es ist dabei erfindungsgemäß vorgesehen, dass der Elektromagnet an und abgeschaltet werden kann, um so zwischen einem Zustand, bei dem das elektromagnetische Feld des Elektromagneten aktiv ist, ein Instrument aufgenommen und gehalten werden kann und einem Zustand, bei dem der Elektromagnet abgeschaltet ist und das elektromagnetische Feld des Elektromagneten nicht aktiv ist, zu wechseln. Der Fachmann wird jedoch unmittelbar anerkennen, dass in einem jeden Zustand, bei dem ein Instrument bestimmungsgemäß aufgenommen oder gehalten werden soll, der Elektromagnet aktiv sein wird, und in einem jeden Zustand, bei dem ein Instrument nicht gehalten werden soll, oder abgelegt werden soll, ausgeschaltet sein kann. Es soll dabei auch verstanden werden, dass bei einem im Folgenden als erfindungsgemäß beschriebenem Abstreifen, der Elektromagnet bevorzugt ausgeschalten ist, dies jedoch ggf. nicht unbedingt notwendig ist.

Erfindungsgemäß ist in der zweiten Position der Elektromagnet aus der Aufnahme ausgefahren. So kann der Elektromagnet, insbesondere bevorzugt in einem angeschalteten Zustand, also mit aktivem elektromagnetischem Feld, besonders leicht über einem Instrument platziert werden. Dies erleichtert das Inkontaktbringen des Elektromagneten mit dem Instrument und erhöht ggf. auch die Präzision, mit der das Instrument durch den Elektromagneten angesprochen und aufgenommen werden kann. Das ist insbesondere der Fall, wenn das Instrument wie zum Beispiel innerhalb eines Sterilgut-Containers oder auf einem chirurgischen Tablett chaotisch gelagert ist.

Es soll verstanden werden, dass die Fähigkeit des erfindungsgemäßen Systems und insbesondere des erfindungsgemäßen Verfahrens, zwischen der ersten und der zweiten Position zu wechseln, die Vielseitigkeit des Greifsystems erhöht und zur Optimierung der sterilen Abläufe beiträgt.

Es wird zudem von einem Fachmann anerkannt werden, dass ein Merkmal, eine Ausführungsform, eine Wirkung oder ein Vorteil, wie hier in Verbindung mit der erfinderischen Vorrichtung und/oder der erfinderischen Umkehrosmoseanlage beschrieben, auch ein Merkmal, eine Ausführungsform, eine Wirkung oder ein Vorteil des erfinderischen Verfahrens sein kann, bzw. umgekehrt.

Es soll im Zusammenhang mit der vorliegenden Erfindung auch verstanden werden, dass das erfindungsgemäße Greifsystem über die Adaptervorrichtung an ein robotisches System, bevorzugt einen Knickarmroboter, angebracht werden kann.

In einer weiteren bevorzugten Ausführungsform des erfindungsgemäßen Verfahrens und/oder des erfindungsgemäßen Greifsystems umfasst das erfindungsgemäße Greifsystem das robotische System, bevorzugt einen Knickarmroboter.

In einer weiteren bevorzugten Ausführungsform des erfindungsgemäßen Verfahrens und/oder des erfindungsgemäßen Greifsystems ist das robotische System in mindestens drei, bevorzugt sechs, Freiheitsgraden beweglich.

In der vorliegenden Beschreibung und den beigefügten Ansprüchen wird, sofern der Kontext nichts anderes erfordert, das Wort "umfassen" und Variationen wie "umfasst" und "umfassend" so verstanden, dass sie die Einbeziehung eines angegebenen Elements, einer ganzen Zahl oder eines Schritts oder einer Gruppe von Elementen, ganzen Zahlen oder Schritten implizieren, jedoch nicht den Ausschluss eines anderen Elements, einer anderen ganzen Zahl oder eines anderen Schritts oder einer anderen Gruppe von Elementen, ganzen Zahlen oder Schritten, obwohl in einigen Ausführungsformen solche anderen Elemente, ganze Zahlen oder Schritte oder Gruppen von Elementen, ganzen Zahlen oder Schritten ausgeschlossen sein können, d.h. der Gegenstand besteht in der Einbeziehung eines angegebenen Elements, einer ganzen Zahl oder eines Schritts oder einer Gruppe von Elementen, ganzen Zahlen oder Schritten.

Die Begriffe "ein" und "eine" und "das" und ähnliche Referenzen, die im Kontext der Beschreibung der Erfindung (insbesondere im Kontext der Ansprüche) verwendet werden, sind so auszulegen, dass sie sowohl den Singular als auch den Plural abdecken, sofern hierin nicht anders angegeben oder durch den Kontext eindeutig widersprochen.

Innerhalb der vorliegenden Anmeldung beziehen sich Begriffe wie "seitlich" oder "lateral", "hinten", "vorne", "oben", "unten", "Boden", "gegenüber", "innen", "außen" oder dergleichen, die die Position eines ersten Objekts relativ zu einem anderen Objekt beschreiben, vorzugsweise auf die relative Position eines jeweiligen Teils oder Objekts in Bezug auf seine Position, wenn es vollständig für seinen vorgesehenen Gebrauch montiert ist.

In einer bevorzugten Ausführungsform des erfindungsgemäßen Verfahrens und/oder der erfindungsgemäßen Vorrichtung ist die Haltevorrichtung derart eingerichtet, dass der Elektromagnet aus der ersten Position und/oder der zweiten Position, in eine dritte Position verfahrbar ist, bei der der Elektromagnet in die Aufnahme zurückgezogen ist.

Das erfindungsgemäße Verfahren umfasst bevorzugt einen Schritt c) des Zurückziehens des Elektromagnets aus der ersten Position und/oder der zweiten Position, in eine dritte Position, bei der der Elektromagnet in die Aufnahme zurückgezogen ist.

Hierdurch wird das Instrument vorteilhaft an der Aufnahme, bzw. dem Konus, abgestreift.

Genauer gesagt kommt durch ein Verfahren des Elektromagneten in die dritte Position, also ein Zurückziehen in die Aufnahme, das gegriffene Instrument vorteilhafterweise in Kontakt mit der Aufnahme und bevorzugt insbesondere mit einem Rand des Konus. Da der Elektromagnet keine magnetische Anziehungskraft mehr ausübt, werden die Instrumente durch die Bewegung und den physischen Kontakt mit der Aufnahme und bevorzugt insbesondere mit einem Rand des Konus abgestreift.

Dies ist insbesondere vorteilhaft, da es eine effiziente und elegante Lösung für das Problem der Restmagnetisierung bietet. Durch das weitere Einfahren des Elektromagneten in den Konus und das Abstreifen der Instrumente am Konus Rand wird ein zusätzlicher Mechanismus zur Entmagnetisierung überflüssig. Dies vereinfacht die Konstruktion des Greifsystems, reduziert die Anzahl der beweglichen Teile und damit auch die Wartungsanforderungen und potenziellen Fehlerquellen. Zudem ermöglicht diese Methode ein schnelles und zuverlässiges Abwerfen der Instrumente, was den gesamten Prozess der Handhabung und Aufbereitung von medizinischen Instrumenten beschleunigt und optimiert. Die Vermeidung von zusätzlichen Abstreifmechanismen wie Wischern trägt außerdem zur Kosteneffizienz, zur Reduzierung der Komplexität des Systems und zu einer verbesserten Handhabung der Hygiene mit Hinblick auf die Vermeidung von Verschleppungen oder Kontaminationen bei.

Der Ausdruck oder Begriff "in die Aufnahme zurückgezogen", wie hierin verwendet, meint dabei bevorzugt eine Positionierung des Elektromagneten und ggf. die mit diesem verbundenen Elemente, wie bspw. einem Positioniermittel, bei der Elektromagnet und ggf. die mit diesem verbundenen Elemente aus einer exponierten oder erweiterten Stellung in eine zurückversetzte oder eingezogene Stellung innerhalb der Aufnahme bewegt wird. Insbesondere ist dabei eine Magnetfläche und/oder Aufnahmefläche des Elektromagneten gegenüber der Aufnahme und insbesondere der Aufnahmekante, bzw. dem Konus und besonders der Konuskante, bezogen auf ein zu greifendes oder gegriffenes Instrument zurückversetzt.

In einer weiteren bevorzugten Ausführungsform des erfindungsgemäßen Verfahrens und/oder des erfindungsgemäßen Greifsystems umfasst die Haltevorrichtung ein Lenkmittel, das dazu eingerichtet und angeordnet ist, den Elektromagneten im Wesentlichen vertikal, bevorzugt im Wesentlichen lotrecht, zu einer Arbeitsoberfläche zu verfahren, wobei bevorzugt das Lenkmittel wenigstens einen biegeweichen Abschnitt umfasst.

In diesem Zusammenhang soll der Begriff "biegeweich" wie hierin verwendet, bevorzugt verstanden werden, als eine Eigenschaft eines Materials oder eines Bauteils, das sich unter Belastung flexibel verformen lässt, ohne dabei seine strukturelle Integrität zu verlieren. Dies bedeutet, dass das Material oder Bauteil in der Lage ist, sich an unterschiedliche Formen und Positionen anzupassen, ohne zu brechen oder dauerhaft deformiert zu werden. Beispiele für biegeweiche Materialien sind Seile, insbesondere Stahlseile, sowie biegeweiche Stäbe, die Gelenke oder flexible Abschnitte enthalten können.

Bevorzugt ist das Lenkmittel ein Seil, bevorzugt Stahlseil, und/oder eine Stange. In diesem Zusammenhang soll verstanden werden, dass damit sich das Positioniermittel an die Instrumente anlegen kann, welche sich aufgrund der chaotischen Lagerung im Siebkorb auch in Schräglage befinden können, das Positioniermittel bevorzugt kippbar ausgestaltet ist. Die Kippbarkeit wird erfindungsgemäß bevorzugt dadurch erreicht, dass das Lenkmittel wenigstens einen biegeweichen Abschnitt umfasst. So kann als Lenkmittel beispielsweise ein Seil verwendet werden. Alternativ können auch andere Lenkmittel eingesetzt werden. Beispielsweise kann das Lenkmittel als zumindest abschnittsweise biegeweicher Stab ausgestaltet sein. Der zumindest abschnittsweise biegeweicher Stab umfasst sodann bspw. als biegeweicher Abschnitt ein Gelenks oder einen anderweitig beigeweichen Abschnitt, bspw. einen Seil- oder Fadenabschnitt.

Dies ist insbesondere vorteilhaft, da durch das Lenkmittel eine präzise und kontrollierte Bewegung des Elektromagneten ermöglicht wird. Die vertikale, vorzugsweise lotrechte Ausrichtung zur Arbeitsoberfläche gewährleistet, dass die Instrumente zunächst direkt und ohne seitliche Verschiebung gehoben oder abgesenkt werden können. Dies minimiert das Risiko von Kollisionen oder Fehlplatzierungen und trägt zu einer erhöhten Genauigkeit bei der Positionierung der Instrumente bei. Die Verwendung eines Seil, bevorzugt Stahlseils oder einer Stange als Lenkmittel bietet zudem eine robuste und langlebige Lösung, die für die präzisen und wiederholbaren Bewegungen innerhalb des Greifsystems erforderlich ist. Darüber hinaus ermöglicht die Konstruktion eine einfache Integration in bestehende robotische Systeme und trägt zur Modularität und Skalierbarkeit des Gesamtsystems bei.

Zusätzlich oder alternativ erlaubt die Verwendung eines Lenkmittels, insbesondere in der Form eines Seil, bevorzugt Stahlseils, auch eine frei schwingende Lagerung des Elektromagneten in einer ausgefahrenen zweiten Position.

Hierbei kann besonders bevorzugt der Elektromagnet in der zweiten Position, bei der der Elektromagnet aus der Aufnahme ausgefahren ist, an dem Lenkmittel insbesondere einem Seil, bevorzugt Stahlseil, frei schwingend gelagert sein. Dies kann insbesondere beim Absenken des Elektromagneten auf das zu greifende Instrument vorteilhaft sein, da durch die frei schwingende Lagerung des Elektromagneten, insbesondere an einem Seil, bevorzugt Stahlseil, in der zweiten Position eine erhöhte Anpassungsfähigkeit des Greifsystems an unterschiedliche Objekt Geometrien ermöglicht wird. Die freie Beweglichkeit des Elektromagneten erlaubt es, dass sich dieser selbstständig in die optimale Position über dem zu greifenden Instrument ausrichten kann, was die Präzision des Greifvorgangs verbessert und das Risiko von Fehlausrichtungen oder Beschädigungen der Instrumente minimiert. Zudem kann die schwingende Lagerung dazu beitragen, die Kräfte, die während des Greifens auf die Instrumente wirken, zu verteilen und somit eine sanftere Handhabung zu gewährleisten.

In Ausführungsformen, bei denen das Lenkmittel ein Seil, bevorzugt Stahlseil, ist, kann vorteilhafterweise der Elektromagnet an einem ersten Ende des Seil, bevorzugt Stahlseils montiert sein, welches mit einem zweiten Ende an der Haltevorrichtung angebracht ist. Insbesondere kann die Haltevorrichtung hierzu eine Führung, bspw. ein Präzisionsrohr, umfassen, welches das Seil, bevorzugt Stahlseil, wenigstens abschnittsweise führt. Das Seil, bevorzugt Stahlseil, kann dabei vorteilhaft wenigstens abschnittsweise innerhalb des Präzisionsrohrs angebracht sein.

Weiterhin vorteilhaft kann die Haltevorrichtung Umlenkungen umfassen, bspw. in Form von Hülsen. So kann das Seil, bevorzugt Stahlseil, vorteilhafterweise auch innerhalb der Greifvorrichtung umgelenkt werden. Bspw. kann mittels einer Umlenkhülse das Seil, bevorzugt Stahlseil, aus einer horizontalen Führung um 90° in eine Vertikale umgelenkt werden.

Der Ausdruck oder Begriff "Arbeitsoberfläche", wie hierin verwendet, meint bevorzugt den Boden des Instrumentenbehälters, auf dem die medizinischen Instrumente abgelegt werden. Diese Oberfläche ist so gestaltet, dass sie eine stabile und sichere Ablage für die Instrumente bietet und gleichzeitig eine einfache Zugänglichkeit für das Greifsystem ermöglicht, um die Instrumente ohne Risiko einer Kontamination oder Beschädigung zu handhaben.

In einer weiteren bevorzugten Ausführungsform des erfindungsgemäßen Verfahrens und/oder des erfindungsgemäßen Greifsystems umfasst die Haltevorrichtung eine Antriebseinheit, insbesondere eine Pneumatik Einheit, die dazu vorgesehen und eingerichtet ist, den Elektromagneten und/oder das Lenkmittel zu bewegen.

Insbesondere kann so auch ein Seil, bevorzugt Stahlseil, als Lenkmittel verwendet werden, um den Elektromagneten anzuheben oder abzusenken und so präzise zwischen den verschiedenen erfindungsgemäßen Positionen zu verfahren.

In einer weiteren bevorzugten Ausführungsform des erfindungsgemäßen Verfahrens und/oder des erfindungsgemäßen Greifsystems umfasst die Haltevorrichtung eine Pneumatik Einheit als Antriebseinheit, und diese umfasst einen Pneumatik Zylinder, der bevorzugt dazu vorgesehen und eingerichtet ist, den Elektromagneten und/oder das Lenkmittel mittels wenigstens zwei verschiedener Druckstufen des Pneumatik Zylinders zu verfahren.

Dies ist insbesondere vorteilhaft, da die Verwendung einer Pneumatik Einheit mit einem Pneumatik Zylinder, der über wenigstens zwei verschiedene Druckstufen verfügt, eine differenzierte und präzise Steuerung des Greifsystems ermöglicht. Die unterschiedlichen Druckstufen erlauben eine fein abgestimmte Bewegung des Elektromagneten und/oder des Lenkmittels, und damit eine verbesserte Verfahrung zwischen den verschiedenen erfindungsgemäßen Positionen des Elektromagneten.

Beispielsweise kann der Elektromagnet in einer ersten Druckstufe in die erste Position gebracht werden, also in die Aufnahme bzw. den äußeren Konus einfahrbar sein, um eine Stabilisierung des Elektromagneten relativ zum gesamten Greifsystem zu ermöglichen, und in einer zweiten Druckstufe, in eine dritte Position weiter in den äußeren Konus einfahrbar sein, um magnetisierbare Instrumente nach dem Abschalten des Elektromagneten abzustreifen.

In einer weiteren bevorzugten Ausführungsform des erfindungsgemäßen Verfahrens und/oder des erfindungsgemäßen Greifsystems umfasst der Elektromagnet ein Positioniermittel. Ein solches Positioniermittel ist insbesondere vorteilhaft, um den Elektromagneten mit Hilfe des Positioniermittels, in Form einer Vorrichtung oder eines Mechanismus, innerhalb der Aufnahme temporär zu stabilisieren. Dies ermöglicht es, den Elektromagneten während des Betriebs in einer definierten Position zu halten, ohne dass er wackelt oder ungewollte Bewegungen ausführt. Dies ist insbesondere vorteilhaft, um eine präzise Handhabung der medizinischen Instrumente zu gewährleisten und gleichzeitig die Flexibilität des Systems zu erhalten.

Dabei ist es bevorzugt, dass das Positioniermittel als Gegenstück zu der Aufnahme ausgebildet ist. Beispielsweise kann das Positioniermittel als Gegenstück zu einem äußeren Konus ausgebildet sein. Dabei kann ein derartiges Positioniermittel als Element zur Führung des Elektromagneten in der Aufnahme und insbesondere beim Einfahren in den äußeren Konus dienen. Es kann bspw. beitragen, dass der Elektromagnet korrekt ausgerichtet und stabil in den Konus einfährt. Weiterhin kann bspw. in einer ersten Druckstufe, wenn der Elektromagnet in die Aufnahme bzw. den äußeren Konus einfährt, das Gegenstück zur Stabilisierung des Elektromagneten beitragen. Es verhindert so vorteilhaft unerwünschte Bewegungen und sorgt für eine sichere Halteposition. Gleichzeitig kann das Gegenstück so geformt sein, dass es die Aufnahmefläche für ein Instrument, insbesondere um eine Aufnahmefläche des Elektromagneten herum, vergrößert, so dass das aufgenommene Instrument stabiler gehalten werden kann. Weiterhin kann, insbesondere in der zweiten Druckstufe das Gegenstück das Abstreifen von Instrumenten unterstützen, die durch Restmagnetisierung noch am Elektromagneten haften. Durch das weitere Einfahren des Elektromagneten in den Konus werden die Instrumente am Rand des Gegenstücks abgestreift.

In einer weiteren bevorzugten Ausführungsform des erfindungsgemäßen Verfahrens und/oder des erfindungsgemäßen Greifsystems ist weiterhin eine Steuereinheit vorgesehen, die konfiguriert ist, Steuerbefehle an die Antriebseinheit zum Verfahren des Elektromagneten zu übermitteln. Eine derartige Steuereinheit kann an verschiedenen Stellen des Greifsystems realisiert werden und insbesondere auch als Teil eines robotischen Systems ausgebildet sein.

Mittels der Steuereinheit werden bevorzugt Steuerbefehle an die Antriebseinheit zum Verfahren des Elektromagneten zu übermittelt, so dass der Elektromagnet zwischen den verschiedenen erfindungsgemäßen Positionen verfahren werden kann. Weiterhin kann die Steuereinheit bevorzugt Steuerbefehle an die Antriebseinheit zum An- oder Abschalten des Elektromagneten übermitteln, so dass der Elektromagnet zwischen einem Zustand, bei dem das elektromagnetische Feld des Elektromagneten aktiv ist, sodass ein Instrument aufgenommen und gehalten werden kann, und einem Zustand, bei dem der Elektromagnet abgeschaltet ist und das elektromagnetische Feld des Elektromagneten nicht aktiv ist, wechseln kann.

Ebenfalls erfindungsgemäß kann, um eine Steuereinheit steuerungstechnisch mit der Haltevorrichtung und insbesondere dem Elektromagneten zu verbinden, die Adaptervorrichtung entsprechende Schnittstellen aufweisen, die dazu eingerichtet und vorgesehen sind, Steuerbefehle zwischen der Steuereinheit und der Haltevorrichtung und insbesondere dem Elektromagneten zu übertragen.

In einer weiteren bevorzugten Ausführungsform des erfindungsgemäßen Verfahrens und/oder des erfindungsgemäßen Greifsystems ist die Steuereinheit dazu eingerichtet, Instrumente in einem leeren Instrumentenbehälter, bevorzugt mittels durch ein optisches System erfassten Bild-Daten, zu erkennen. In einer bevorzugten Ausführungsform umfasst das erfindungsgemäße Greifsystem ein optisches System, das insbesondere dazu eingerichtet ist, Informationen über den Instrumentenbehälter und ggf. dessen Bestückung mit Instrumenten zu detektieren. Ein solches optisches System kann hierzu optische Sensoren, insbesondere eine Kamera umfassen. Das optische System und/oder die Steuereinheit kann dabei vorteilhaft so eingerichtet sein, dass eine Auswertung der gesammelten Sensordaten des optischen Systems, zu einer Überprüfung verwendet werden, ob alle Instrumente gegriffen wurden und/oder ob Instrumente in dem Instrumentenbehälter vorhanden sind.

Hierbei wird das Greifsystem und insbesondere die Steuereinheit und/oder das optische System bevorzugt auf ein Erkennen eines leeren Instrumentenbehälters, insbesondere eines Siebkorbes, trainiert. Die Steuereinheit kann hierzu vorteilhaft ein neuronales Netzwerk umfassen und/oder mit einem solchen neuronalen Netzwerk in Kommunikationsverbindung stehen. Insbesondere ist ein solches neuronales Netzwerk vorteilhaft bevorzugt auf ein Erkennen eines leeren Instrumentenbehälters, insbesondere eines Siebkorbes, trainiert. Entsprechend kann das erfindungsgemäße Verfahren und/oder das erfindungsgemäße Greifsystem umfassen, dass in einem Fall, indem beim Abfahren und/oder optischen Scannen in verschiedenen Bereichen noch Instrumente detektiert werden, diese mit dem Greifsystem derart angefahren werden, dass sich der Elektromagnet vertikal über einem der Instrumente befindet. Sodann, können diese Instrumente mittels des erfindungsgemäßen Greifsystems einzeln und nacheinander entnommen werden, bis sie alle entnommen wurden. Ein Fachmann wird dabei unmittelbar erkennen, dass dieses erfindungsgemäße Verfahren eine Abkehr vom Stand der Technik darstellt, in dem im Wesentlichen entsprechende Systeme auf das Vorhandensein von Instrumenten trainiert werden.

Das erfindungsgemäße Verfahren umfasst bevorzugt einen Schritt des Abfahrens und/oder optischen Scannens mittels des optischen Systems, wobei der Instrumentenbehälter abgefahren und ggf. vorhandene Instrumente, bevorzugt wie oben beschrieben, detektiert werden. Weiter umfasst ein Schritt des Abfahrens und/oder optischen Scannens mittels des optischen Systems ein translatorisches Abfahren und/oder translatorisches optisches Scannen des Instrumentenbehälters in einem dreidimensionalen Raster.

In einer weiteren bevorzugten Ausführungsform des erfindungsgemäßen Verfahrens und/oder des erfindungsgemäßen Greifsystems umfasst die Haltevorrichtung weiter wenigstens eine Druckfeder, die bevorzugt so eingerichtet und angeordnet ist, dass die Druckfeder durch das Eigengewicht des Elektromagneten und ggf. weiterer Komponenten, insbesondere der mit dem Elektromagneten verbundenen Komponenten, insbesondere eines Positioniermittels, komprimiert wird. Eine solche Druckfeder ist bevorzugt in Ihrer Kompressionsrichtung horizontal, d.h. bevorzugt orthogonal zu der Hauptgreifrichtung des Elektromagneten angeordnet.

Beispielsweise kann bei einer Entlastung des Lenkmittels, insbesondere in der Form eines Seil, bevorzugt Stahlseils, durch das Aufsetzen des Elektromagneten auf den Boden des Instrumentenbehälters und/oder ein Instrument diese Druckfeder so angeordnet und eingerichtet sein, dass sie durch das Eigengewicht des Elektromagneten und aller weiteren Komponenten komprimiert wird. Bei einem Aufsetzen des Elektromagneten und einer Entlastung des Seils, bevorzugt Stahlseils, dehnt sich die Druckfeder dann aus und führt somit das Seil, bevorzugt Stahlseil, innerhalb der Haltevorrichtung, und insbesondere, wenn vorhanden, einer Führung des Seils, bevorzugt Stahlseils, bspw. eines Rohrs, insbesondere Präzisionsrohrs. Dadurch wird vermieden, dass sich das Seil, bevorzugt Stahlseil, durch die Entlastung in der Haltevorrichtung, insbesondere einer Führung des Seils, bevorzugt Stahlseils, bspw. eines Rohrs oder einer ggf. vorhandenen Umlenkung verklemmt.

In einer weiteren bevorzugten Ausführungsform des erfindungsgemäßen Verfahrens und/oder des erfindungsgemäßen Greifsystems weist die Haltevorrichtung wenigstens eine Positionierfeder auf, die angeordnet und eingerichtet ist, den Elektromagneten in der Aufnahme zu positionieren.

In einer weiteren bevorzugten Ausführungsform des erfindungsgemäßen Verfahrens und/oder des erfindungsgemäßen Verfahrens Greifsystems ist die Haltevorrichtung derart eingerichtet, dass der Elektromagnet, bevorzugt aus der dritten Position, in eine vierte Position, bei der der Elektromagnet in einen Einzugskanal des Positioniermittels hineingezogen ist, verfahrbar ist.

Insbesondere kann ein erfindungsgemäßes Verfahren einen Schritt umfassen, wobei der Elektromagnet, bevorzugt aus der dritten Position, in eine vierte Position, in einen Einzugskanal des Positioniermittels hineingezogen wird.

Dabei kann gleichsam ein Pneumatik Zylinder bevorzugt dazu vorgesehen und eingerichtet sein, den Elektromagneten und/oder das Lenkmittel mittels wenigstens einer weiteren Druckstufe des Pneumatik Zylinders in eine solche vierte Position zu verfahren.

Insbesondere kann zwischen dem Elektromagneten und ggf. dem Positioniermittel eine weitere Feder angeordnet sein, welche um das Lenkmittel, insbesondere das Seil, bevorzugt Stahlseil, herum angeordnet ist. Diese weitere Feder hat bevorzugt eine höhere Federkonstante als die Positionierfeder. Die Feder ist bevorzugt zwischen Magnet und oberem Ende des Positioniermittels, insbesondere oberem Ende des Einzugskanals des Positioniermittels angeordnet. In einer weiteren bevorzugten Ausführungsform ist der Einzugskanal an seinem zur Instrumentenaufnahme vorgesehenen Ende teilweise, bevorzugt vollständig mit einer Abdeckung verschlossen. Dies ermöglicht besonders vorteilhaft das Abstreifen von Instrumenten, insbesondere spitzen Instrumententeilen, die ggf. andernfalls am Magnet hängend in den Einzugskanal gezogen würden, ohne abgestreift zu werden.

Ein erfindungsgemäßes Anfahren der vierten Position ist insbesondere dann vorteilhaft, wenn das Abstreifen von Instrumenten in einer dritten Position nicht erfolgreich war. Gleichzeitig oder alternativ kann das Verfahren des Elektromagneten in die vierte Position gleichzeitig oder anstelle des Verfahrens des Elektromagneten in die dritte Position erfolgen. Mit anderen Worten wird dann das Greifsystem derart eingerichtet, dass der Elektromagnet sich sogleich zum Abstreifen in den Einzugskanal zurückziehen kann.

Das Zurückziehen des Elektromagneten in den Einzugskanal ist insbesondere vorteilhaft, wenn ein Instrument mit der Instrumentenspritze, d.h. im Wesentlichen in vertikaler Ausrichtung zum Instrumentenbehälterboden, an dem Elektromagneten hängt und somit beim Einfahren des Elektromagneten in eine dritte Position, also bevorzugt in die Aufnahme, nicht in Kontakt mit der Aufnahme gelangt.

In einer weiteren bevorzugten Ausführungsform umfasst das erfindungsgemäße Greifsystem einen Kraft- bzw. Dehnungssensor, der dazu eingerichtet ist, ein missglücktes Abstreifen eines Instruments in der dritten Position zu detektieren. Wahlweise kann gleichzeitig oder alternativ ein missglücktes Abstreifen optisch, insbesondere durch das optische System, detektiert werden. Alternativ kann die vierte Position jeweils nach einen Schritt des Zurückziehens des Elektromagneten aus der ersten Position und/oder der zweiten Position in eine dritte Position, bei der der Elektromagnet in die Aufnahme (11) zurückgezogen ist, wodurch das Instrument abgestreift wird, durchgeführt werden. In diesem Fall könnte wahlweise auf eine entsprechende Sensorik verzichtet werden.

Wird nun also die vierte Position angefahren und der Druck erhöht, bewegt sich der Elektromagnet in dem Positioniermittel, insbesondere den Einzugskanal, nach oben und komprimiert dabei die weitere Feder.

An der Unterseite des Positioniermittels kann bevorzugt eine relativ dünne aber biegesteife Abdeckung vorgesehen sein, die den Elektromagneten nach unten bedeckt und vorzugsweise selbst weder magnetisch noch magnetisierbar ist. Das anhaftende Instrument wird durch die Abdeckung daran gehindert, dem Elektromagnet in das Positioniermittel bzw. den Einzugskanal zu folgen. Vorteilhaft werden so Elektromagnet und Instrument voneinander mechanisch getrennt, sodass sich das Instrument von dem Elektromagneten und dem Positioniermittel löst und auf diese Weise abgelegt wird.

In einer weiteren bevorzugten Ausführungsform des erfindungsgemäßen Greifsystems, umfasst das Greifsystem eine Schutzzelle. Eine solche Schutzzelle kann insbesondere die Anlagensicherheit des erfindungsgemäßen Greifsystems erhöhen. Dabei wird bevorzugt das gesamte Greifsystem, in einer entsprechenden Schutzzelle, beispielsweise bestehend aus Aluminiumprofilen und PC-Platten mit entsprechenden Durchbrüchen für Förderbänder oder weiterer Schnittstellen, untergebracht. Des Weiteren können in der Schutzzelle Durchbrüche zur Vermeidung von Unfällen und unerlaubten Eingriffen mit Lichtschranken und sonstigen Schutzmechanismen vorgesehen werden.

In einer weiteren bevorzugten Ausführungsform des erfindungsgemäßen Greifsystems, umfasst das Greifsystem einen Sauggreifer. Der Sauggreifer kann ergänzend ausgerüstet werden, um das Greifen von Schalen oder nicht magnetisierbaren Instrumenten mit glatten Oberflächen zu ermöglichen.

### Kurzbeschreibung der Figuren

Die vorliegende Erfindung wird im Folgenden ausführlicher erläutert unter Bezugnahme auf die Zeichnungen, aus denen weitere Merkmale, Ausführungsformen und Vorteile entnommen werden können. In den in den Abbildungen gezeigten Ausführungsformen sind Elemente, die ähnliche oder identische Funktionen haben, mit gleichen Bezugszeichen versehen. Es wird darauf hingewiesen, dass die Abbildungen möglicherweise nicht maßstabsgetreu zueinander sind.

Dabei zeigt:
FIG 1 eine schematische Darstellung eines Greifsystems gemäß einer ersten erfindungsgemäßen Ausführungsform;
FIG 2 eine schematische Schnittzeichnung eines Greifsystems gemäß der ersten erfindungsgemäßen Ausführungsform in einer zweiten Position;
FIG 3 eine schematische Schnittzeichnung eines Greifsystems gemäß der ersten erfindungsgemäßen Ausführungsform in einer Zentrierposition; und
FIG 4 eine schematische Schnittzeichnung eines Greifsystems gemäß der ersten erfindungsgemäßen Ausführungsform in einer dritten Position.
FIG 5A und 5B zeigen schematische Schnittzeichnung eines Greifsystems gemäß der ersten erfindungsgemäßen Ausführungsform in einer dritten (Fig. 5A) und in einer vierten (Fig. 5B) Position.

### Detaillierte Beschreibung

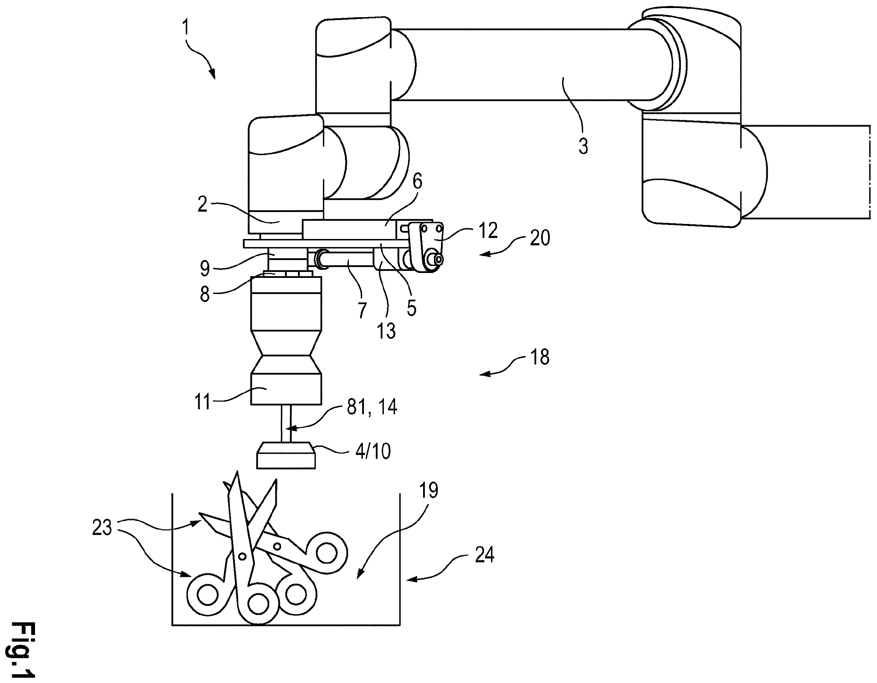

Figur 1 zeigt eine schematische Darstellung des Greifsystems gemäß einer ersten erfindungsgemäßen Ausführungsform. Dabei ist das Greifsystem zum Greifen und Positionieren von medizinischen Instrumenten 23 geeignet. In Figur 1 ist dargestellt, wie mittels einer Adaptervorrichtung 2 die Greifvorrichtung 1 an einem robotischen System 3, hier einem 6 DOF Roboterarm, angebracht ist. Hierdurch ist vorteilhafterweise das Greifsystem in seiner Gesamtheit in mindestens drei translatorischen Freiheitsgraden, alternativ aber in 6 DOF mithilfe eines Knickarmroboters 3, automatisiert steuerbar verfahrbar.

Die Greifvorrichtung 1 umfasst den Elektromagneten 4 zur Aufnahme von magnetisierbaren Instrumenten aus einem Instrumentenbehälter. Hierzu ist der Elektromagnet in der Haltevorrichtung 18 vorgesehen, wobei der Elektromagnet 4 mittels dieser Haltevorrichtung 18 auch an der Adaptervorrichtung 2 und damit am Steuersystem, hier einem robotischen System 3, angebracht ist. Die Haltevorrichtung 18 hat eine Aufnahme 11 in Form eines Konus 11, in der der Elektromagnet 4 aufnehmbar ist.

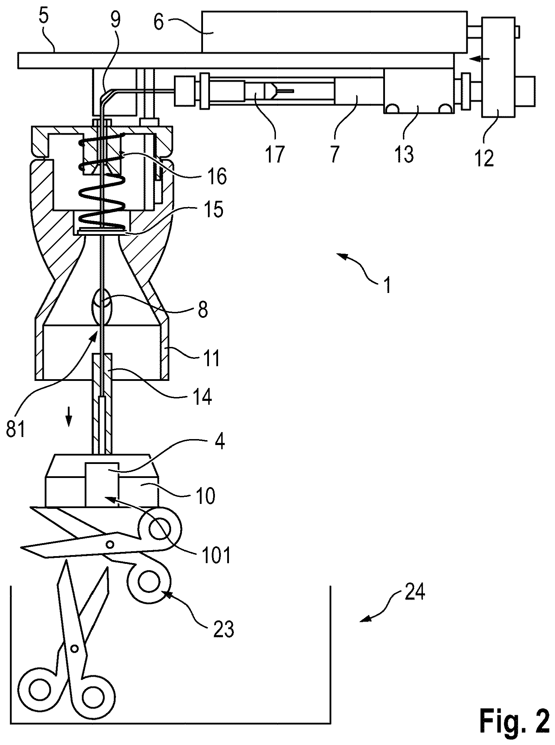

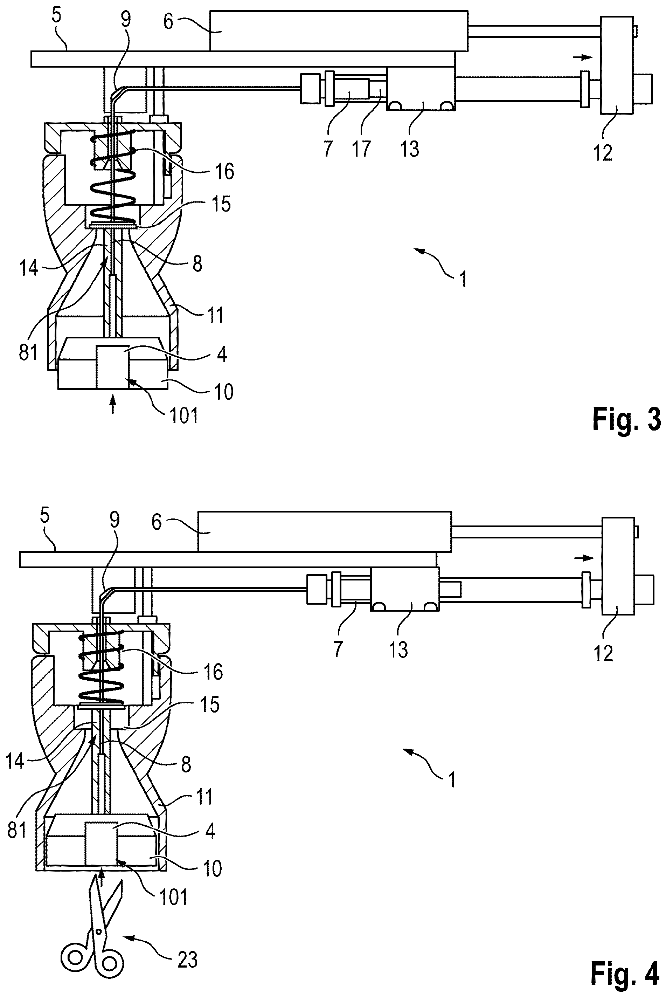

Figur 1 zeigt die Greifvorrichtung in einer zweiten Position, bei der der Elektromagnet 4 aus der Aufnahme 11 ausgefahren ist. Erfindungsgemäß, ist die Haltevorrichtung 18 derart eingerichtet, dass der Elektromagnet 4 aus der dargestellten zweiten Position in die erste Position, bei der der Elektromagnet 4 in der Aufnahme 11 aufgenommen ist, verfahrbar ist. Ebenfalls kann erfindungsgemäß der Elektromagnet 4 aus der ersten Position, bei der der Elektromagnet 4 in der Aufnahme 11 aufgenommen ist (vgl. Fig. 3, in der diese erste Position noch nicht vollständig erreicht ist), in die zweite Position, bei der der Elektromagnet 4 aus der Aufnahme 11 ausgefahren ist (Fig. 1 und Fig 2), verfahren werden. In dieser zweiten Position kann der Elektromagnet 4 mit der geforderten Nachgiebigkeit Instrumente 23 aus dem Siebkorb 24 heraus aufnehmen.

Figur 1 zeigt den Zusammenbau des Greifsystems umfassend das robotische System 3, den Adapter 2 und die Haltevorrichtung 18 mit Elektromagnet 4. In der dargestellten Position kann der Elektromagnet 4 mittels des erfindungsgemäßen Verfahrens Instrumente aus dem Instrumentenbehälter entnehmen.

Die Haltevorrichtung 18 weist als Lenkmittel 8 sowohl das Stahlseil 81 als auch die Stange 14 auf, so dass das Lenkmittel wenigstens einen biegeweichen Abschnitt umfasst. Die Lenkmittel 8 erlauben es, den Elektromagneten 4 im Wesentlichen vertikal, bevorzugt im Wesentlichen lotrecht, zu einer Arbeitsoberfläche 19 zu verfahren. Das Seil 81, bevorzugt ein Stahlseil, hat einen Durchmesser um 0,5 mm bis 1 mm und ist mit einem, dem Stand der Technik entsprechenden, linearen Aktor mit mindestens einem Freiheitsgrad verbunden.

Das Stromversorgungskabel für den Elektromagneten 4 ist vorzugsweise entlang des Seils 81 verlegt und bevorzugt verstärkt um Knickungen zu vermeiden. Der Aktor ist mit der Basis des Greifers verbunden. Das Seil 81 bzw. der Elektromagnet 4 ist im herabgelassen Zustand, also in der zweiten Position, bei der der Elektromagnet 4 aus der Aufnahme 11 ausgefahren ist, an dem Lenkmittel 8 insbesondere dem Stahlseil 81 frei schwingend gelagert, um ein querkräftefreies Aufsetzen auf die Instrumente 23 zu ermöglichen, ohne eine Kollision bzw. ein Verkanten durch weitere chaotisch angeordnete Instrumente 23 bzw. Hinterschnitte im Siebkorb 24 zu verursachen.

In Figur 2 ist eine schematische Schnittzeichnung des Greifsystems gemäß der ersten erfindungsgemäßen Ausführungsform in einer zweiten Position gezeigt und Figur 3 zeigt eine schematische Schnittzeichnung des Greifsystems gemäß der ersten erfindungsgemäßen Ausführungsform in einer Zentrierposition, kurz bevor die erste Position vollständig erreicht ist.

Die Haltevorrichtung 18 erlaubt ebenfalls, den Elektromagneten 4 aus der ersten Position und/oder der zweiten Position in eine dritte Position zu verfahren, welche in Fig. 4 dargestellt ist. Diese Figur 4 zeigt eine schematische Schnittzeichnung des Greifsystems gemäß der ersten erfindungsgemäßen Ausführungsform in einer dritten Position, bei der der Elektromagnet 4 in die Aufnahme 11 zurückgezogen ist. Die Pfeile zeigen jeweils die Bewegungsrichtung der Bauteile an.

Mittels der Lenkmittel 8, nämlich dem Stahlseil 81 und der Stange 14, kann der Elektromagnet 4 im Wesentlichen vertikal und lotrecht zur Arbeitsoberfläche 19 verfahren werden. Hierzu umfasst die Haltevorrichtung 18 eine Antriebseinheit 20 in Form einer Pneumatik Einheit 20. Da das Seil 81 zum einen mit dem Elektromagneten 4 an einem ersten Ende des Seils 81 verbunden ist und mit seinem zweiten Ende an der Haltevorrichtung 18, insbesondere mit der Pneumatik Einheit 20, verbunden ist, kann der Elektromagnet 4 mittels der Pneumatik Einheit 20 bewegt werden. Auf der Oberseite der Adapterplatte 5 ist der doppeltwirkende Pneumatik Zylinder 6 mit einem Hub von vorzugsweise 100 mm montiert. Über einen Adapter am Pneumatik Zylinder 6 wird ein Präzisionsrohr 7 in einem Gleitlager 13 mit der Ausfahrbewegung des Pneumatik Zylinders 6 bewegt. Das innerhalb des Präzisionsrohrs 7 angebrachte Seil 81, bevorzugt ein Stahlseil, wird über eine Außenhülse 9 von vorzugsweise 90° umgelenkt. An dem Ende des Seils 81, bevorzugt Stahlseils, ist der Elektromagnet 4 mit einem Gegenstück 10 zu dem äußeren Konus 11, als Positioniermittel 10, montiert.

Die Pneumatik Einheit 20 fungiert dabei als Antriebseinheit 20 und umfasst den Pneumatik Zylinder 6, der den Elektromagneten 4 und das Seil 81 mittels der verschiedenen bevorzugt voreingestellten Druckstufen des Pneumatik Zylinders 6 verfährt. Wie in Figur 3 dargestellt, kann der Elektromagnet 4 in der Aufnahme 11 hier im äußeren Konus eingefahren werden, wobei der Pneumatik Zylinder 6 in der ersten Druckstufe betrieben wird. Um mit den am Elektromagneten 4 anliegenden bzw. aus dem Siebkorb 23 entnommenen Instrumenten 23 sicher und definiert räumlich d.h. in 6DOF verfahren zu können, wird erfindungsgemäß vorgeschlagen, den Elektromagneten 4 in der ersten Position, bei der der Elektromagnet 4 in der Aufnahme 11, also den äußeren Konus 11, eingefahren ist, aufzunehmen (siehe Fig. 3). Dies ermöglicht eine Stabilisierung des Elektromagneten 4 relativ zum gesamten Greifsystem. Lediglich eine Rotation entlang der Hochachse des vorzugsweisen runden Elektromagneten 4 ist in dieser Konfiguration noch möglich.

In Figur 4 ist die erfindungsgemäße Greifvorrichtung 1 in einer dritten Position, der Abwurfposition für Instrumente 23, dargestellt.

Da die magnetisierbaren Instrumente 12 teilweise eine Restmagnetisierung nach dem Kontakt mit dem elektromagnetischen Feld des Elektromagneten 4 aufweisen können, wird vorgeschlagen, diese nach dem Abschalten des Elektromagneten 4 zusätzlich abzustreifen. Dies kann nötig sein, wenn, wie eingangs beschrieben, eine Restmagnetisierung vorhanden ist oder der Elektromagnet 4 im Dauer-an Betrieb betrieben wird.

Es soll verstanden werden, dass selbst wenn der Elektromagnet 4 abgeschaltet wird, insbesondere bei leichten Instrumenten 23, wie bspw. Pinzetten, die Restmagnetisierung ausreichen kann, um ein gezieltes Abwerfen bzw. -legen der Instrumente 23 zu verhindern. Es wird deshalb erfindungsgemäß vorgeschlagen, das Abstreifen bevorzugt ohne zusätzliche Mechanismen wie Wischer oder anders geartete aktive Abstreifer zu realisieren, indem der Elektromagnet 4 weiter in den äußeren Konus 11 in die dritte Position einfährt und die Instrumente 12 durch den Konus Rand vom sich entfernenden Elektromagneten 4 gelöst werden (siehe Fig. 4).

Ein erfindungsgemäßes zweistufiges Einfahren des Elektromagneten 4 in eine erste bzw. dritte Position in die Aufnahme 11, insbesondere den äußeren Konus 11, wird vorzugsweise pneumatisch über zwei Druckstufen des Pneumatik Zylinders 6 realisiert. Alternativ ist eine mechatronische Ausgestaltung der erfindungsgemäßen Greifvorrichtung ebenfalls möglich.

Dabei ist in der dargestellten Ausführungsform des erfindungsgemäßen Greifsystems die im Folgenden beschriebene Realisierung gewählt. Die Oberkante einer Stange 14, an welcher der Elektromagnet 4 befestigt ist, schlägt bei einer Aufwärtsbewegung, während eines Ausfahrens des Pneumatik Zylinders 6, an die Federgrundplatte 15 an. Die Bewegungsrichtung ist durch die dargestellten Pfeile in den Figuren 2, 3, und 4 symbolisiert. In der ersten Druckstufe und der in Fig. 3 dargestellten ersten Position, wird der Pneumatik Zylinder 6 mit einem geringen Druck betrieben, sodass sich unmittelbar nach Kontakt mit der Positionierfeder 16 ein Kräftegleichgewicht einstellt. Der Elektromagnet 4 befindet sich damit in der Zentrierposition. Die Unterkante des Elektromagneten 4 ist dabei noch nicht vollständig in den äußeren Konus 11 eingefahren und die erste Position noch nicht vollständig erreicht. In der zweiten Druckstufe des Pneumatik Zylinders 6 wird der Druck erhöht und die Feder 16 weiter komprimiert, sodass der Elektromagnet 4 vollständig in den äußeren Konus 11 eintaucht und die erste Position vollständig erreicht wird. Die Feder 16 ist so ausgelegt, dass diese bei maximaler Kraft des Pneumatik Zylinders 6, vorzugsweise 10 bar, vollständig komprimiert wird und in einer dritten Position der Elektromagnet 4 in die Aufnahme 11 zurückgezogen ist (Fig. 4). Alle überstehenden Instrumente 23 bzgl. der Unterseite des Elektromagneten 4 werden dabei an der Kante des äußeren Konus 11 abgestreift.

Bei einer Entlastung des Seils 81 durch das Aufsetzen des Elektromagneten 4 auf einen Instrumentenbehälterboden 19 oder ein Instrument 23 ist in horizontaler Lage eine weitere Druckfeder 17 angebracht. Diese Feder 17 ist so ausgelegt, dass sie durch das Eigengewicht des Elektromagneten 4 und aller weiteren Komponenten, insbesondere des Gegenstücks 10, komprimiert wird. Bei einem Aufsetzen des Elektromagneten 4 und einer Entlastung des Seils 81 dehnt sich die Feder 17 und führt somit das Seil 81 innerhalb des Präzisionsrohrs 7. Dadurch wird vermieden, dass das Seil 81 schlaff wird und sich z.B. durch die Entlastung in der Umlenkung 9 oder innerhalb des Präzisionsrohrs 7 verklemmt.

Grundsätzlich soll durch diesen Aufbau das Ansteuern des Pneumatik Zylinders 6 in zwei verschiedenen Druckstufen bei der Ausfahrbewegung sowie das Aus- und Einfahren des doppeltwirkenden Pneumatik Zylinders 6 erreicht werden. Damit die Aus- und Einfahrbewegung nicht ruckartig erfolgt, wird vor den Eingängen des Pneumatik Zylinders 6 bevorzugt jeweils ein Drosselventil (nicht dargestellt) angebracht. Die Bewegungsrichtung des doppeltwirkenden Pneumatik Zylinders 6 wird vorzugsweise über ein 5/2-Wegeventil mit Federrückstellung gesteuert. Das elektromagnetisch ansteuerbare Wegeventil kann mit einem der konfigurierbaren Ausgänge des robotischen Systems 3 verbunden und über das Roboterprogramm gesteuert werden.

Das 5/2-Wegeventil wird bevorzugt so angeordnet und eingerichtet, dass der Pneumatik Zylinder 6 in der durch die Feder 17 rückgestellten Lage des Wegeventils eingefahren ist. Sobald der Ausgang des Roboters 2 schaltet, beginnt die Ausfahrbewegung des Pneumatik Zylinders 6 und der Elektromagnet 4 wird mit dem gegriffenen Instrument 23 in den äußeren Konus 11 gezogen. Damit das Instrument 23 nicht direkt abstreift, indem der Elektromagnet 4 zu weit in den äußeren Konus 11 gezogen wird, erfolgt die Ausfahrbewegung des Pneumatik Zylinders 6 in zwei Druckstufen.

Diese werden bevorzugt über ein weiteres 5/2-Wegeventil gesteuert. Die zwei möglichen Positionen des 5/2-Wegeventils schalten zwischen zwei verschiedenen Weg-Optionen mit einem hohen Druckniveau (vorzugsweise 2 bar) und einem geringen Druckniveau (vorzugsweise 0,5 bar). Ein Druckminderer zu Beginn der ersten Option mit niedrigem Niveau sorgt vorzugsweise für einen gegenüber dem hohen Niveau reduzierten Druck bei Eintritt der Druckluft in das System. In der umgekehrten Stellung des Wegeventils liegt der hohe Druck an. Um einen kurzzeitigen Druckabfall aufgrund des zusätzlichen Volumens (des nicht geschalteten Weges) zu verhindern, werden vorzugsweise zwei Rückschlagventile an der erforderlichen Position integriert. Das 5/2-Wegeventil wird ebenfalls über einen konfigurierbaren Ausgang des robotischen Systems gesteuert.

Die Haltevorrichtung 18 des erfindungsgemäßen Greifsystems kann insbesondere derart eingerichtet sein, dass der Elektromagnet 4, bevorzugt aus der dritten Position, in eine vierte Position, bei der der Elektromagnet 4 in einen Einzugskanal 101 des Positioniermittels 10 hineingezogen ist, verfahrbar ist. Dabei wird vorzugsweise der Pneumatik Zylinder 6 mit weiteren Druckstufe versehen.

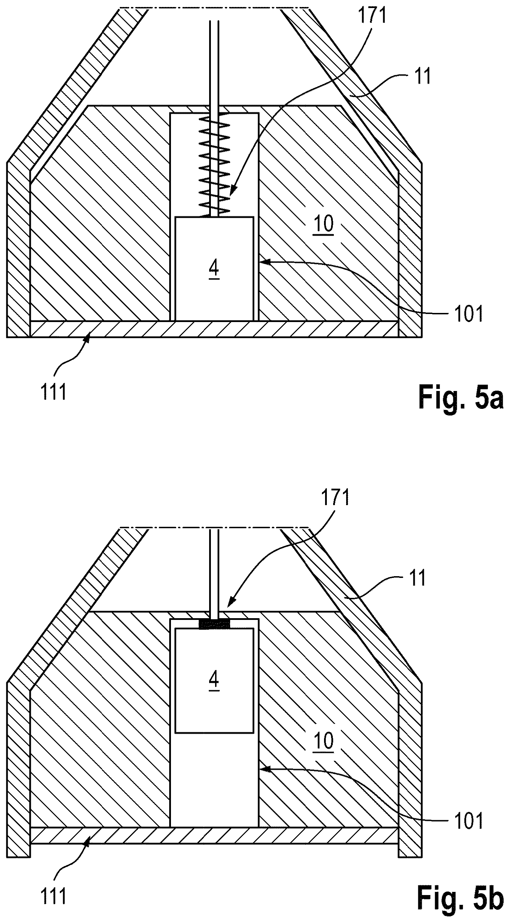

Wie in Fig. 5A und 5B zu sehen, ist hierzu zwischen dem Elektromagneten 4 und dessen Positioniermittel 10, ggf. ausgebildet als absenkbares Gehäuse 10, eine weitere Feder 171 um das Seil 81 herum angeordnet. Diese Feder 171 hat bevorzugt eine höhere Federkonstante als die Positionierfeder 16. Die Feder 171 ist zwischen Magnet 4 und oberem Ende des Positioniermittels 10, insbesondere dem oberem Ende des Einzugskanals 101 des Positioniermittels 10 angeordnet. Zudem ist der Einzugskanal 101 an seinem zur Instrumentenaufnahme vorgesehenen Ende mit einer Abdeckung 111, hier vollständig, verschlossen. Das Positioniermittel 10 kann dabei mit der Abdeckung 111 einteilig, als integrales Bauteil, oder mehrteilig, bspw. als Kappe, gefertigt sein.

Die weitere Druckstufe in der vierten Position kommt zum Einsatz, wenn das Abstreifen von Instrumenten 23 in der dritten Position nicht erfolgreich war. Dies kann passieren, wenn ein Instrument 23 mit der Instrumentenspritze (also quasi vertikal) an dem Elektromagneten 4 hängt, wie beispielhaft in Fig. 4 dargestellt, und somit beim Einfahren des Elektromagneten 4 in den äußeren Konus 11 nicht in Kontakt mit dem äußeren Konus 11 gelangt. Wird nun also aus der auch in Fig. 5A dargestellten dritten Position, die in Fig. 5B dargestellte vierte Position angefahren und der Druck gegenüber der dritten Position erhöht, bewegt sich der Elektromagnet 4 von der in Fig. 5A dargestellten Position in dem Einzugskanal 101 des Positioniermittels 10 nach oben (siehe Fig. 5B) und komprimiert dabei die weitere Feder 171. Dabei entfernt sich der Elektromagnet 4 von dem restmagnetisierten Instrument, welches von dem Elektromagnet 4 durch die Abdeckung 111 getrennt ist, wodurch dieses abgelegt wird. Das Positioniermittel 10 gelangt dabei entweder mit Erreichen der dritten Druckstufe oder beim Übergang der dritten Druckstufe in die vierte Druckstufe in Anlage mit der Aufnahme 11, bevorzugt im Bereich des äußeren Konus 11.

### Referenzzeichenliste

1- Greifvorrichtung
2- Adaptervorrichtung, Roboterinterface
3- Robotisches System, insbesondere 6DOF Knickarm-Roboter
4- Elektromagnet
5- Adapterplatte
6- Pneumatik Zylinder
7- Präzisionsrohr
8- Lenkmittel
81-Seil, bevorzugt Stahlseil,
9- Außenhülse, Umlenkung
10-Positioniermittel, Gegenstück
101- Einzugskanal
11-Aufnahme, äußerer Konus
12-Adapter
13-Gleitlager
14-Stange
15-Federgrundplatte
16-Positionierfeder
17-Druckfeder
18-Haltevorrichtung
19-Arbeitsoberfläche
20-Antriebseinheit
21- Optisches System
22- Steuereinheit
23-Instrument
24-Instrumentenbehälter
171- weitere Feder

## Patentansprüche

1. Greifsystem zum Greifen und Positionieren von medizinischen Instrumenten (23), umfassend:
a) eine Greifvorrichtung (1), die einen Elektromagneten (4) umfasst und zur Aufnahme von magnetisierbaren Instrumenten (23) aus einem Instrumentenbehälter (24) mittels des Elektromagneten (4) eingerichtet und vorgesehen ist;
b) eine Adaptervorrichtung (2), die dazu eingerichtet und vorgesehen ist, die Greifvorrichtung (1) an einem Steuersystem (3), bevorzugt einem robotischen System (3), anzubringen;
c) eine Haltevorrichtung (18), wobei der Elektromagnet (4) mittels der Haltevorrichtung (18) an der Adaptervorrichtung (2) angebracht ist;
d) wobei die Haltevorrichtung (18) eine Aufnahme (11), insbesondere einen Konus (11), zur Aufnahme des Elektromagneten (4) umfasst;
und
wobei die Haltevorrichtung (18) derart eingerichtet ist, dass der Elektromagnet (4) zwischen einer ersten Position, bei der der Elektromagnet (4) in der Aufnahme (11) aufgenommen ist und einer zweiten Position, bei der der Elektromagnet (4) aus der Aufnahme (11) ausgefahren ist, verfahrbar ist.

2. Greifsystem (1) nach Anspruch 1, wobei die Haltevorrichtung (18) derart eingerichtet ist, dass der Elektromagnet (4) aus der ersten Position und/oder der zweiten Position in eine dritte Position verfahrbar ist, bei der der Elektromagnet (4) in die Aufnahme (11) zurückgezogen ist.

3. Greifsystem (1) nach einem der vorhergehenden Ansprüche, wobei die Haltevorrichtung (18) ein Lenkmittel (8) umfasst, das dazu eingerichtet und angeordnet ist, den Elektromagneten (4) im Wesentlichen vertikal, bevorzugt im Wesentlichen lotrecht, zu einer Arbeitsoberfläche (19) zu verfahren, wobei bevorzugt das Lenkmittel (8) wenigstens einen biegeweichen Abschnitt umfasst.

4. Greifsystem (1) nach einem der vorhergehenden Ansprüche, wobei die Haltevorrichtung (18) eine Antriebseinheit (20), insbesondere eine Pneumatik Einheit (20) umfasst, die dazu vorgesehen und eingerichtet ist, den Elektromagneten (4) und/oder das Lenkmittel (8) zu bewegen.

5. Greifsystem (1) nach einem der vorhergehenden Ansprüche, bevorzugt Anspruch 5, wobei die Haltevorrichtung (18) eine Pneumatik Einheit (20) als Antriebseinheit (20) umfasst, die einen Pneumatik Zylinder (6) umfasst, der bevorzugt dazu vorgesehen und eingerichtet ist, den Elektromagneten (4) und/oder das Lenkmittel (8) mittels wenigstens zwei verschiedener Druckstufen des Pneumatik Zylinders (6) zu verfahren.

6. Greifsystem (1) nach einem der vorhergehenden Ansprüche, wobei der Elektromagnet (4) ein Positioniermittel (10) umfasst.

7. Greifsystem (1) nach einem der vorhergehenden Ansprüche, weiter umfassend eine Steuereinheit (22), die konfiguriert ist, Steuerbefehle an die Antriebseinheit (20) zum Verfahren des Elektromagneten (4) zu übermitteln.

8. Greifsystem (1) nach einem der vorhergehenden Ansprüche, wobei die Steuereinheit (22) dazu eingerichtet ist, Instrumente (23) in einem leeren Instrumentenbehälter (24), bevorzugt mittels durch ein optisches System (21) erfassten Bild-Daten, zu erkennen.

9. Greifsystem (1) nach einem der vorhergehenden Ansprüche, wobei der Elektromagnet (4) in der zweiten Position, bei der der Elektromagnet (4) aus der Aufnahme (11) ausgefahren ist, an dem Lenkmittel (8) insbesondere einem Seil (81), bevorzugt Stahlseil (81), frei schwingend gelagert ist.

10. Greifsystem (1) nach einem der vorhergehenden Ansprüche, wobei die Haltevorrichtung (18) weiter wenigstens eine Druckfeder (17) umfasst, die bevorzugt so eingerichtet und angeordnet ist, dass die Druckfeder (17) durch das Eigengewicht des Elektromagneten (4) komprimiert wird.

11. Greifsystem (1) nach einem der vorhergehenden Ansprüche, wobei die Haltevorrichtung (18) wenigstens eine Positionierfeder (16) aufweist, die angeordnet und eingerichtet ist, den Elektromagneten (4) in der Aufnahme (11) zu positionieren.

12. Greifsystem (1) nach einem der vorhergehenden Ansprüche, wobei die Haltevorrichtung (18) derart eingerichtet ist, dass der Elektromagnet (4), bevorzugt aus der dritten Position, in eine vierte Position, bei der der Elektromagnet (4) in einen Einzugskanal (101) des Positioniermittels (10) hineingezogen ist, verfahrbar ist.

13. Verfahren zur Entnahme von chirurgischen Instrumenten (23) aus einem Instrumentenbehälter (24), insbesondere einem Siebkorb (24), und insbesondere zur Vereinzelung und Handhabung der chirurgischen Instrumente (23) umfassend
einen Schritt des Bereitstellens eines Greifsystems (1); und
wahlweise, einen Schritt a) des Aktivierens des Elektromagneten (4);
einen Schritt b) des Verfahrens des Elektromagnets (4) aus einer ersten Position, bei der der Elektromagnet (4) in der Aufnahme (11) aufgenommen ist, in eine zweite Position, bei der der Elektromagnet (4) aus der Aufnahme (11) ausgefahren ist;
einen Schritt c) des Aufnehmens eines magnetisierbaren Instruments (23) mittels des Elektromagneten (4) in der zweiten Position;
einen Schritt d) des Verfahrens des Elektromagnets (4) aus der zweiten Position, bei der der Elektromagnet (4) aus der Aufnahme (11) ausgefahren ist, in die erste Position, bei der der Elektromagnet (4) in der Aufnahme (11) aufgenommen;
wahlweise, einen Schritt e) des Deaktivierens des Elektromagnets (4);
wobei das Greifsystem (1) bevorzugt ein Greifsystem (1) nach einem der vorhergehenden Ansprüche ist.

14. Verfahren nach Anspruch 13, weiter umfassend,
einen Schritt des Zurückziehens des Elektromagnets (4) aus der ersten Position und/oder der zweiten Position, in eine dritte Position, bei der der Elektromagnet (4) in die Aufnahme (11) zurückgezogen ist, wodurch das Instrument (23) abgestreift wird.

15. Verfahren nach Anspruch 13 oder Anspruch 14, weiter umfassend einen Schritt wobei der Elektromagnet (4), bevorzugt aus der dritten Position, in eine vierte Position, in einen Einzugskanal (101) des Positioniermittels (10) hineingezogen wird.
